# EUROPEAN PATENT SPECIFICATION

(11) **EP 2 146 684 B1**
(45) Date of publication and mention of the grant of the patent: **08.12.2010**
(21) Application number: 08750162.3
(22) Date of filing: 07.05.2008
(51) Int. Cl.: A61K 8/31, A61Q 19/00, A61K 8/86

(54) **COSMETIC COMPOSITIONS WITH ETHOXYLATED URETHANE**
KOSMETISCHE ZUSAMMENSETZUNGEN MIT ETHOXYLIERTEM URETHAN
COMPOSITIONS COSMÉTIQUES AVEC DE L'URÉTHANE ÉTHOXYLÉ

(30) Priority: 21.05.2007 US 939154 P
(43) Date of publication of application: 27.01.2010
(73) Proprietor: Unilever PLC, London EC4Y 0DY (GB); Unilever N.V., 3013 AL Rotterdam (NL)
(72) Inventor: ROSE, Walter, New Haven, Connecticut 06511 (US); MINER, Philip, Edward, Trumbull, Connecticut 06611 (US)
(74) Representative: Acham, Nicholas Clive
(86) International application number: PCT/EP2008/055646
(87) International publication number: WO 2008/141942

(56) References cited:
- US-A- 5 972 324
- US-A1- 2005 106 193
- US-A1- 2006 127 344
- US-A1- 2007 048 240

## Description

### BACKGROUND OF THE INVENTION

The invention relates to cosmetic compositions whose major component other than water is petrolatum incorporating additives achieving a product with good consistency to allow for a smooth aesthetic application onto human skin.

Petrolatum is one of the oldest skin treatment products still in commerce today. For over 100 years, the Chesebrough Company and its successors have sold the substance under the brand Vaseline®. There is good reason for the longevity of this product. Its occlusive and healing properties render this product especially efficacious against dry and damaged skin.

Of course there are many drawbacks to petrolatum. This substance is greasy. When in contact with clothes or anything else, the product has a tendency to be transferred through rub off. An approach to this problem is found in US 5 407 678 (Rose et al.) which employs aluminum starch octenylsuccinate and a C₁₂-C₁₅ alkyl lactate to provide a non-greasy yet efficacious product active against dry skin.

A non-greasy and easily spreadable composition of petrolatum is reported in US 5 595 745 (Znaiden et al.). The petroleum butter mentioned in this patent utilizes C₁₈-C₃₀ acyl lactylate as a facilitator to achieve improved skin healing, moisturization and other skin benefit properties.

US 2006/127344, US2007/48240, US 2005/106193 disclose the combination of petrolatum with an ethoxylated urethane in aqueous cosmetic compositions.

Despite these advances, problems remain. Traditional petrolatum has a rather stiff consistency. Pure petrolatum is not readily spreadable onto the skin surface. Thus there remains the problem of finding formulations easily rubbable onto skin.

### SUMMARY OF THE INVENTION

A cosmetic composition is provided that includes:
(i) from 1 to 40% by weight of petrolatum;
(ii) from 20 to 90% by weight of water; and
(iii) from 0.085 to 0.4% by weight of an ethoxylated urethane
(iv) from 0.001 to 10% by weight of cocoa butter

### DETAILED DESCRIPTION OF THE INVENTION

Now it has been found that cosmetic compositions with substantial amounts of petrolatum can have their aesthetic properties improved through incorporation of an ethoxylated urethane. Not only is product stability improved but the ethoxylated urethane achieves a thickening effect without being sticky or tacky. Further the resultant thickened composition is still easily rubbable into skin.

Amounts of the petrolatum for purposes of this invention may range from 1% to 40%, preferably from 5 to 30%, more preferably from 8 to 15% by weight of the composition.

Water will also be present in compositions of this invention. Amounts of the water may range from 20 to 90%, preferably from 30 to 80%, and optimally from 50 to 65% by weight of the composition.

An essential further component of compositions according to the present invention is that of an ethoxylated urethane.

Ethoxylated urethanes of the present invention that are particularly preferred are dimers such as those according to the structure below. 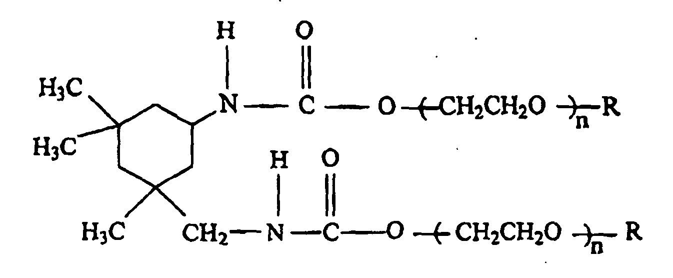 wherein n is a whole number from about 50 to 120, preferably 75-100, and most preferably 75 or 100, and R is independently a C₁₂-C₂₄ alkyl or alkenyl group, preferably an alkyl group, more preferably a C₁₈ alkyl group.

Particularly preferred are ethoxylated urethanes available commercially under the tradename Dermothix 75 (INCI Name: Disteareth-75 IPDI) and Dermothix 100 (INCI Name: Disteareth-100 IPDI) and their combinations, sold by Alzo International Incorporated (Sayreville, New Jersey).

Another component which is present in compositions of this invention is cocoa butter. Amounts of cocoa butter range from 0.001 to 10%, preferably from 0.1 to 10%, and optimally from 0.8 to 2% by weight of the composition.

The term "cocoa butter" is also defined as oleum theobromatis (theobroma oil). This material is obtained from the cacao bean by expression, decoction or extraction by solvent. Particularly common is a production method wherein cacao seeds are compressed between hot or cold plates. Typical properties are a specific gravity ranging from 0.858 to 0.864 (100/25°C), melting point between 30 to 35°C, refractive index (n40/D) of 1.4537 to 1.4585; saponification number 188 to 200 and an iodine number from 32 to 43.

Another useful component of the present invention is that of a taurate polymer or copolymer. A particularly preferred copolymer is one wherein the taurate repeating monomer unit is acryloyl dimethyltaurate (in either free acid or salt form). Monomers forming the copolymer with taurate may include: styrene, hydroxyethyl acrylic acid, hydroxyethyl methacrylic acid, acrylic acid, methacrylic acid, vinyl chloride, vinyl acetate, vinyl pyrrolidone, isoprene, vinyl alcohol, vinyl methylether, chloro-styrene, dialkylamino-styrene, maleic acid, acrylamide, methacrylamide and mixtures thereof. Where the term "acid" appears, the term means not only the free acid but also C₁-C₃₀ alkyl esters, anhydrides and salts thereof. Preferably but not exclusively the salts may be ammonium, alkanolammonium, alkali metal and alkaline earth metal salts.

Most preferred as the copolymer is a sodium acrylate/sodium acryloyldimethyl taurate copolymer sold as Simulgel EG® by Seppic Corporation. Other taurate copolymers may also be useful and can include Simulgel NS® which is known by its INCl name of Hydroxyethyl Acrylate/Sodium Acryloyldimethyl Taurate Copolymer, also from Seppic Corporation.

Number average molecular weight of taurate polymers according to the invention may range from 1,000 to 3,000,000, preferably from 3,000 to 100,000, optimally from 10,000 to 80,000.

Amounts of the taurate polymer when present may range from 0.001 to 10%, preferably from 0.01 to 8%, more preferably from 0.1 to 5%, optimally from 0.2 to 2% by weight of the composition.

Emollient materials may serve as cosmetically acceptable carriers. These may be in the form of natural or synthetic esters, hydrocarbons and silicones. Amounts of the emollients may range anywhere from 0.1 to 60%, preferably between 1 and 30% by weight of the composition.

Among the ester emollients are:
(a) Alkyl esters of saturated fatty acids having 10 to 24 carbon atoms. Examples thereof include behenyl neopentanoate, isononyl isonanonoate, isopropyl myristate and octyl stearate.
(b) Ether-esters such as fatty acid esters of ethoxylated saturated fatty alcohols.
(c) Polyhydric alcohol esters. Ethylene glycol mono- and di-fatty acid esters, diethylene glycol mono- and di-fatty acid esters, polyethylene glycol (200-6000) mono- and di-fatty acid esters, propylene glycol mono- and di-fatty acid esters, polypropylene glycol 2000 monostearate, ethoxylated propylene glycol monostearate, glyceryl mono- and di-fatty acid esters, polyglycerol poly-fatty esters, ethoxylated glyceryl mono-stearate, 1,3-butylene glycol monostearate, 1,3-butylene glycol distearate, polyoxyethylene polyol fatty acid ester, sorbitan fatty acid esters, and polyoxyethylene sorbitan fatty acid esters are satisfactory polyhydric alcohol esters. Particularly useful are pentaerythritol, trimethylolpropane and neopentyl glycol esters of C₁-C₃₀ alcohols.
(d) Wax esters such as beeswax, spermaceti wax and tribehenin wax.
(e) Sugar ester of fatty acids such as sucrose polybehenate and sucrose polycottonseedate.

Natural ester emollients principally are based upon mono-, di- and tri- glycerides. Representative glycerides include sunflower seed oil, cottonseed oil, borage oil, borage seed oil, primrose oil, castor and hydrogenated castor oils, rice bran oil, soybean oil, olive oil, safflower oil, shea butter, jojoba oil and combinations thereof. Animal derived emollients are represented by lanolin oil and lanolin derivatives. Amounts of the natural esters may range from 0.1 to 20% by weight of the composition.

Hydrocarbons which are suitable cosmetically acceptable carriers include mineral oil, C₁₁-C₁₃ isoparaffins, polybutenes, and especially isohexadecane, available commercially as Permethyl 101A (Presperse Incorporated). Hydrogenated polyisobutenes such as Panalane H300E (Ineos Corporation) are useful both as carriers and adjunct thickeners. The hydrocarbons and particularly hydrogenated polyisobutenes may be present in amounts from 0.01 to 20%, preferably from 0.1 to 5%, and optimally from 0.5 to 3% by weight of the composition.

Fatty acids having from 10 to 30 carbon atoms may also be suitable as cosmetically acceptable carriers. Illustrative of this category are pelargonic, lauric, myristic, palmitic, stearic, isostearic, oleic, linoleic, linolenic, hydroxystearic and behenic acids.

A wide variety of silicones including materials of liquid, solid or semi-solid consistency at room temperature can be useful as emollients for this invention. Liquid silicones include silicone oils which may be divided into the volatile and non-volatile variety. The term "volatile" as used herein refers to those materials which have a measurable vapor pressure at ambient temperature. Volatile silicone oils are preferably chosen from cyclic (cyclomethicone) or linear polydimethylsiloxanes containing from 3 to 9, preferably from 4 to 5, silicon atoms. Commercially available volatile silicone oils include DC 200, DC 244, DC 245, DC 344 and DC 345, all supplied by the Dow Corning Corporation; SF-1204, SF-1202 Silicone Fluids, GE 7207 and GE 7158 sourced from GE Silicones; and SWS-03314 sourced from SWS Silicones Corporation.

Useful non-volatile silicone oils include polyalkyl siloxanes, polyalkylaryl siloxanes and polyether siloxane copolymers. The essentially non-volatile polyalkyl siloxanes useful herein include, for example, polydimethyl siloxanes with viscosities of from about 5 x 10⁻⁶ to 0.1 m²/s at 25°C. Among the preferred non-volatile emollients useful in the present compositions are the polydimethyl siloxanes having viscosities from 1 x 10⁻⁵ to 4 x 10⁻⁴ m²/s at 25°C. Representative commercial materials include polyalkyl siloxanes sold under the Viscasil Series from G.E. Silicones, and the DC 200 series sold by the Dow Corning Corporation. Polyalkylaryl siloxanes including polymethylphenyl siloxanes such as SF 1075 methyl-phenyl fluid and 556 Cosmetic Grade Fluid (Dow Corning Corporation) may also be useful. Illustrative polyoxyalkylene ether copolymers are commercially available as SF 1066 (G.E. Silicones) and PEG-10 Dimethicone (Shin-Etsu).

Another class of non-volatile silicones are emulsifying and non-emulsifying silicone elastomers. Representative of this category is dimethicone/vinyl dimethicone crosspolymer available as Dow Corning 9040, General Electric SFE 839, and Shin-Etsu KSG-18. Silicone waxes such as Silwax WS-L (dimethicone copolyol laurate) may also be useful.

Amounts of the silicone may range from 0.05 to 50%, preferably from 0.5 to 40%, more preferably from 2 to 20%, optimally from 5 to 12% by weight of the composition.

Emulsifiers may also be present in compositions of this invention. Total concentration of the emulsifier when present may range from 0.1 to 10%, preferably from 0.5 to 2%, optimally from 1.8 to 12% by weight of the composition. The emulsifier may be selected from the group consisting of anionic, nonionic, cationic and amphoteric actives. Particularly preferred nonionic emulsifiers are those with a C₁₀-C₂₀ fatty alcohol or acid hydrophobe condensed with from 2 to 100 moles of ethylene oxide or propylene oxide per mole of hydrophobe; C₂-C₁₀ alkyl phenols condensed with from 2 to 20 moles of alkylene oxide; mono- and di-fatty acid esters of ethylene glycol; fatty acid monoglyceride; sorbitan, mono- and di- C₈-C₂₀ fatty acids; and polyoxyethylene sorbitan as well as combinations-thereof. Alkyl polyglycosides and saccharide fatty amides (e.g. methyl gluconamides) and trialkylamine oxides are also suitable nonionic emulsifiers.

Preferred anionic emulsifiers include soap, alkyl ether sulfates and sulfonates, alkyl sulfates and sulfonates, alkylbenzene sulfonates, alkyl and dialkyl sulfosuccinates, C₈-C₂₀ acyl isethionates, C₈-C₂₀ alkyl ether phosphates, C₈-C₂₀ sarcosinates, C₈-C₂₀ acyl lactylates, sulfoacetates and combinations thereof.

Useful amphoteric surfactants include cocoamidopropyl betaine, C₁₂-C₂₀ trialkyl betaines, sodium lauroamphoacetate and sodium laurodiamphoacetate.

Adjunct humectants may be employed in the present invention. These are generally polyhydric alcohol-type materials. Typical polyhydric alcohols include glycerin, propylene glycol, dipropylene glycol, polypropylene glycol, polyethylene glycol, sorbitol, hydroxypropyl sorbitol, hexylene glycol, 1,3-butylene glycol, isoprene glycol, 1,2,6-hexanetriol, ethoxylated glycerol, propoxylated glycerol and mixtures thereof. The amount of adjunct humectant may range anywhere from 0.2 to 40%, preferably between 1 and 25%, most preferably between 2 and 15% by weight of the composition. Most preferred is glycerin as an adjunct humectant or moisturizer.

Sunscreen agents may also be included in compositions of the present invention. Particularly preferred are such materials as ethylhexyl p-methoxycinnamate (Parsol MCX®), Avobenzene (Parsol 1789®), and benzophenone-3 (also known as Oxybenzone). Inorganic sunscreen actives may be employed such as microfine (1 to 100 nm) titanium dioxide and zinc oxide. Amounts of the sunscreen agents when present may generally range from 0.1 to 30%, preferably from 2 to 20%, optimally from 4 to 10% by weight of the composition.

Preservatives can desirably be incorporated into the compositions of this invention to protect against the growth of potentially harmful microorganisms. Particularly preferred preservatives are phenoxyethanol, methyl paraben, propyl paraben, imidazolidinyl urea, dimethyloldimethylhydantoin, ethylenediaminetetraacetic acid salts (EDTA), sodium dehydroacetate, methylchloroisothiazolinone, methylisothiazolinone, iodopropynbutylcarbamate and benzyl alcohol. The preservatives should be selected having regard for the use of the composition and possible incompatibilities between the preservatives and other ingredients. Preservatives are preferably employed in amounts ranging from 0.0001% to 2% by weight of the composition.

Compositions of the present invention-may include vitamins. Illustrative vitamins are vitamin A (retinol), vitamin B₂, vitamin B₃ (niacinamide), vitamin B₆, vitamin C, vitamin E, folic acid and biotin. Derivatives of the vitamins may also be employed. For instance, vitamin C derivatives include ascorbyl tetraisopalmitate, magnesium ascorbyl phosphate and ascorbyl glycoside. Derivatives of vitamin E include tocopheryl acetate, tocopheryl palmitate and tocopheryl linoleate. DL-panthenol and derivatives may also be employed. Total amount of vitamins when present in compositions according to the present invention may range from 0.001 to 10%, preferably from 0.01% to 1%, optimally from 0.1 to 0.5% by weight of the composition.

Another type of useful substance can be that of an enzyme such as amylases, oxidases, proteases, lipases, cellulases, elastases and combinations.

Skin lightening compounds may be included in the compositions of the invention. Illustrative substances are placental extract, lactic acid, niacinamide, arbutin, kojic acid, ferulic acid, Pisum Sativum (Actiwhite LS 9808, ex Cognis), resorcinol and derivatives including 4-substituted resorcinols and combinations thereof. Amounts of these agents may range from 0.1 to 10%, preferably from 0.5 to 2% by weight of the composition.

Sunless tanners may also be formulated with compositions of this invention. Representative of this category is dihydroxyacetone, erythrulose, Troxerutin, melanin, mahkanni and mixtures thereof. Adjunct agents include amino acids, peptides, amines and combinations. Amounts of the sunless tanner may range from 0.1 to 15%, preferably from 0.5 to 10%, optimally from 1 to 5% by weight.

Desquamation promoters may be present. Illustrative are the alpha-hydroxycarboxylic acids and beta-hydroxycarboxylic acids. The term "acid" is meant to include not only the free acid but also salts and C₁-C₃₀ alkyl or aryl esters thereof and lactones generated from removal of water to form cyclic or linear lactone structures. Representative acids are glycolic, lactic and malic acids. Salicylic acid is representative of the beta-hydroxycarboxylic acids. Amounts of these materials when present may range from 0.01 to 15% by weight of the composition.

A variety of herbal extracts may optionally be included in compositions of this invention. The extracts may either be water soluble or water-insoluble carried in a solvent which respectively is hydrophilic or hydrophobic. Water and ethanol are the preferred extract solvents. Illustrative extracts include those from green tea, chamomile, licorice, aloe vera, grape seed, citrus unshui, willow bark, sage, thyme and rosemary.

Also included may be such materials as lipoic acid, retinoxytrimethylsilane (available from Clariant Corporation under the Silcare 1 M-75 trademark), dehydroepiandrosterone (DHEA) and combinations thereof. Ceramides (including Ceramide 1, Ceramide 3, Ceramide 3B and Ceramide 6) as well as pseudoceramides may also be useful. Amounts of these materials may range from 0.000001 to 10%, preferably from 0.0001 to 1% by weight of the composition.

Colorants, opacifiers and abrasives may also be included in compositions of the present invention. Each of these substances may range from 0.05 to 5%, preferably between 0.1 and 3% by weight of the composition.

The viscosity of compositions according to the present invention may range from 2,000 to 1 million cps, preferably from 50,000 to 900,000 cps, optimally from 600,000 to 800,000 cps. The optimal products are often referred to as "butters".

The term "comprising" is meant not to be limiting to any subsequently stated elements but rather to encompass non-specified elements of major or minor functional importance. In other words the listed steps, elements or options need not be exhaustive. Whenever the words "including" or "having" are used, these terms are meant to be equivalent to "comprising" as defined above.

It should be noted that in specifying any range of concentration or amount, any particular upper concentration can be associated with any particular lower concentration or amount.

The following examples will more fully illustrate the embodiments of this invention. All parts, percentages and proportions referred to herein and in the appended claims are by weight unless otherwise illustrated.

### EXAMPLE 1

Cocoa butter lotions of the present invention are presented in table 1.

**TABLE 1**

| INGREDIENT | Sample (weight %) | | | | | | | |
|---|---|---|---|---|---|---|---|---|
| | 1 | 2 | 3 | 4 | 5 | 6 | 7 | 8 |
| Stearic acid | 2.5 | 3.5 | 3.5 | 3.5 | 3.5 | 3.5 | 2.5 | 1.5 |
| Glycerol monostearate/stearamide AMP | 1.5 | 0.5 | 0.5 | 1.5 | 1.5 | 0.5 | 1.5 | 2.5 |
| Glycerol monostearate | 0.7 | 1.2 | 1.2 | 1.2 | 1.2 | 0.7 | 1.5 | 1.5 |
| Cetyl alcohol | 0.4 | 0.6 | 0.6 | 0.8 | 0.8 | 0.8 | 0.8 | 0.6 |
| Dimethicone | 3.0 | 6.0 | 6.0 | 6.0 | 6.0 | 3.0 | 1.0 | 1.0 |
| Petrolatum | 9.0 | 10.0 | 15.0 | 20.0 | 10.0 | 10.0 | 15.0 | 15.0 |
| Cetiol MS® | 3.0 | 4.0 | 4.0 | 2.0 | 3.0 | 2.0 | 4.5 | 4.5 |
| Cocoa butter | 0.9 | 1.5 | 1.5 | 1.5 | 0.1 | 0.1 | 4.0 | 0.1 |
| Microcrystalline wax | 0.25 | 0.25 | 0.25 | 0.25 | 0.25 | 0.25 | 0.25 | 0.25 |
| Paraffin wax | 0.5 | 0.5 | 0.5 | 0.5 | 0.5 | 0.5 | 0.5 | 0.5 |
| Hydrogenated polyisobutene | 2.5 | 1.5 | 0.5 | 0.5 | 0.5 | 2.5 | 1.5 | 1.5 |
| Disteareth-75 IPDI | 0.075 | 0.050 | 0.050 | 0.050 | 0.065 | 0.065 | 0.085 | 0.085 |
| Propyl paraben | 0.1 | 0.1 | 0.1 | 0.1 | 0.1 | 0.1 | 0.1 | 0.1 |
| Shea butter | 0.1 | 0.1 | 0.1 | 0.1 | 0.1 | 0.1 | 0.1 | 0.1 |
| Disodium EDTA | 0.05 | 0.05 | 0.05 | 0.05 | 0.05 | 0.05 | 0.05 | 0.05 |
| Methyl paraben | 0.2 | 0.2 | 0.2 | 0.2 | 0.2 | 0.2 | 0.2 | 0.2 |
| PEG-90 diisostearate | 0.5 | 0.5 | 0.5 | 0.5 | 0.5 | 0.5 | 0.5 | 0.5 |
| Simulgel EG® | 1.5 | 0.5 | 2.5 | 2.5 | 2.5 | 1.5 | 1.5 | 1.5 |
| Triethanolamine (85% Active) | 0.8 | 0.3 | 1.4 | 1.4 | 1.4 | 0.8 | 0.8 | 0.8 |
| Xanthan gum | 0.05 | 0.05 | 0.05 | 0.05 | 0.05 | 0.05 | 0.05 | 0.05 |
| Glycerin | 6.9 | 6.9 | 6.9 | 6.9 | 6.9 | 6.9 | 6.9 | 6.9 |
| Cyclopentasiloxane | 4.0 | 4.0 | 4.0 | 4.0 | 4.0 | 4.0 | 4.0 | 4.0 |
| Potato starch | 0.7 | 1.0 | 1.0 | 1.0 | 1.2 | 1.2 | 2.0 | 2.0 |
| DC 1501 (cyclopentasiloxane & dimethiconol) | 0.5 | 1.5 | 1.5 | 1.5 | 1.5 | 1.5 | 0.5 | 1.0 |
| Phenoxyethanol | 0.4 | 0.4 | 0.4 | 0.4 | 0.4 | 0.4 | 0.4 | 0.4 |
| Ethylene brassylate | 0.15 | 0.20 | 0.20 | 0.30 | 0.30 | 0.01 | 0.01 | 0.01 |
| Water | Qs | Qs | Qs | Qs | Qs | Qs | Qs | Qs |

### EXAMPLE 2

A series of comparative experiments were conducted to evaluate ethoxylated urethane and other materials on their effects toward producing a product with good consistency and smooth aesthetic application onto skin (stick/slip friction).

### Procedure

The procedure measured friction during the first 10 minutes of drying after application onto a surface. The test is conducted in an environmentally controlled chamber at 21 °C and 20% relative humidity. Sample size of 100 microliters is spread on a Lucite table over a 6 by 1.25 inch area (2.54 cm to the inch). The table is attached to the cross-head of an Instron Model 4501 Materials Testing System. A 3 by 1 inch 15 gram aluminum sled covered with a 100% rayon nonwoven is pulled across the same area at a rate of 10 cm/min, starting one minute after application and repeated each minute for ten minutes. The integral of force versus a distance of 40 mm (i.e. amount of work of dynamic slip friction units of gram-mm) is calculated for each of the intervals. Also measured is the static load (grams) or stick. Three runs are conducted for each product

### Formulas and Results

A series of samples were prepared for the evaluations. The formulas are recorded in table 2.

**TABLE 2**

| INGREDIENT | Sample (weight %) | | | | | | | | | |
|---|---|---|---|---|---|---|---|---|---|---|
| | 1 (Ref.) | 2 | 3 | 4 (Ref.) | 5 | 6 (Ref.) | 7 (Ref.) | 8 (Ref.) | 9 (Ref.) | 10 (Ref.) |
| Petrolatum | 9.0 | 9.0 | 9.0 | 9.0 | 1.0 | -- | 9.0 | -- | -- | -- |
| Hydrogenated polyisobutene | -- | -- | -- | -- | -- | -- | -- | 1.0 | 1.5 | 2.0 |
| Cocoa butter | 0.9 | 0.9 | 0.9 | 0.9 | 0.9 | 0.9 | -- | -- | -- | -- |
| Disteareth-75 IPDI | 0.075 | 0.15 | 0.3 | 0.5 | 0.15 | 0.15 | 0.15 | 0.075 | 0.075 | 0.075 |
| Water | Qs | Qs | Qs | Qs | Qs | Qs | Qs | Qs | Qs | Qs |

| | | | | | | | | | | |
|---|---|---|---|---|---|---|---|---|---|---|
| Ref. = not according to the invention | | | | | | | | | | |

Results of the Instron tests are recorded in table 3. Lower values for stick indicate less stickiness (tack) of the sample. Stickiness (tack) is an undesirable skinfeel. Likewise lower values of friction indicate improved feel aesthetics.

**TABLE 3**

| Sample | Stick or Static Load (Grams) | Dynamic Friction or Work (Gram-mm) |
|---|---|---|
| 1 | 34.2 | 1573.2 |
| 2 | 25.9 | 1198.5 |
| 3 | 27.3 | 1345.8 |
| 4 | 35.0 | 1707.8 |
| 5 | 24.4 | 1382.8 |
| 6 | 28.2 | 1301.0 |
| 7 | 32.1 | 1561.8 |
| 8 | 42.2 | 1829.0 |
| 9 | 37.1 | 1705.3 |
| 10 | 30.1 | 1445.2 |

Samples 1-4 demonstrate that the most frictionless performance for the ethoxylated urethane peaked around 0.15%. Levels between 0.085 and 0.4% were within an operative range. Small amounts of cocoa butter were also useful as shown by comparison of sample 2 with sample 7. Likewise, performance was increased by the presence of hydrogenated polyisobutene as reflected in samples 8-10.

## Claims

1. A cosmetic composition comprising:
(i) from 1 to 40% by weight of petrolatum;
(ii) from 20 to 90% by weight of water;
(iii) from 0.085 to 0.4% by weight of an ethoxylated urethane; and
(iv) from 0.001 to 10% by weight of cocoa butter.

2. The composition according to claim 1 wherein the ethoxylated urethane is a dimer according to the following structure: 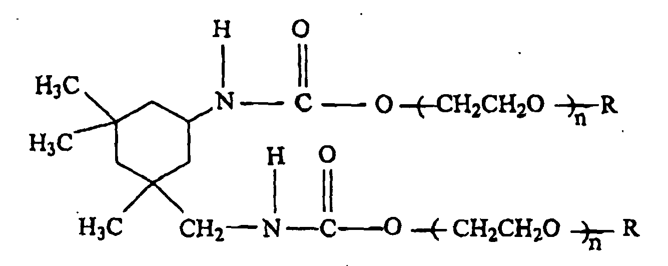 wherein n is a whole number from 50 to 120 and R is independently a C₁₂-C₂₄ alkyl or alkenyl group.

3. The composition according to claim 2 wherein the ethoxylated urethane has R which is a C₁₈ alkyl group and n ranges from 75 to 100.

4. The composition according to any one of the preceding claims further comprising from 0.1 to 10% by weight of a taurate polymer.

5. The composition according to claim 4 wherein the taurate polymer is sodium hydroxyethyl acrylate/sodium acryloyldimethyl taurate.

6. The composition according to claim 4 wherein the taurate polymer is sodium acrylate/sodium acryloyldimethyl taurate.

7. The composition according to any one of the preceding claims further comprising from about 0.1 to 5% by weight of hydrogenated polyisobutene.

## Patentansprüche

1. Eine kosmetische Zusammensetzung, umfassend
(i) von 1 bis 40 Gew.-% Petrolatum,
(ii) von 20 bis 90 Gew.-% Wasser,
(iii) von 0,085 bis 0,4 Gew.-% ethoxyliertes Urethan und
(iv) von 0,001 bis 10 Gew.-% Kakaobutter.

2. Zusammensetzung nach Anspruch 1, worin das ethoxylierte Urethan ein Dimer entsprechend der folgenden Struktur ist 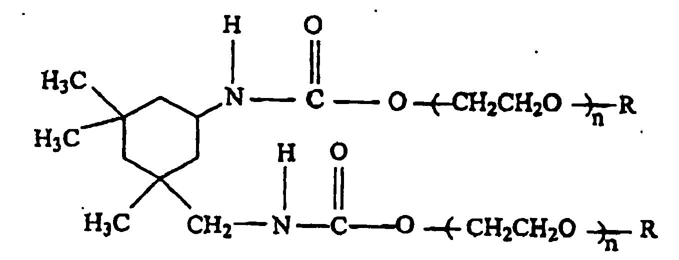 worin n eine ganze Zahl von 50 bis 120 und R unabhängig voneinander eine C₁₂-C₂₄-Alkyl- oder -Alkenyl-Gruppe ist.

3. Zusammensetzung nach Anspruch 2, worin das ethoxylierte Urethan ein R aufweist, das eine C₁₈-Alkyl-Gruppe darstellt und wobei n zwischen 75 bis 100 variiert.

4. Zusammensetzung nach irgendeinem der vorhergehenden Ansprüche, die des Weiteren 0,1 bis 10 Gew.-% eines Taurat-Polymers enthält.

5. Zusammensetzung nach Anspruch 4, worin das Taurat-Polymer Natriumhydroxyethylacrylat/Natriumacryloyldimethyl-Taurat ist.

6. Zusammensetzung nach Anspruch 4, worin das Taurat-Polymer ein Natriumacrylat/Natriumacryloyldimethyl-Taurat ist.

7. Zusammensetzung nach irgendeinem der vorhergehenden Ansprüche, die des Weiteren etwa 0,1 bis 5 Gew.-% hydriertes Polyisobuten enthält.

## Revendications

1. Composition cosmétique comprenant :
(i) de 1 à 40 % en poids de pétrolatum ;
(ii) de 20 à 90 % en poids d'eau ;
(iii) de 0,085 à 0,4 % en poids d'un uréthane éthoxylé ; et
(iv) de 0,001 à 10 % en poids de beurre de cacao.

2. Composition selon la revendication 1, dans laquelle l'uréthane éthoxylé est un dimère selon la structure suivante : 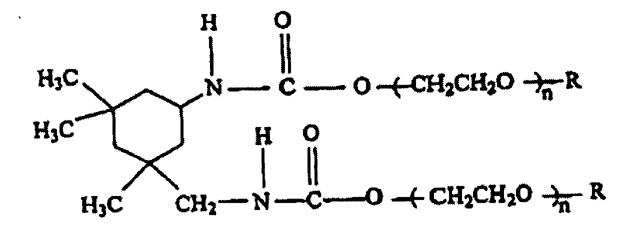 dans laquelle n est un nombre entier de 50 à 120 et R représente indépendamment un groupe alkyle ou alcényle en C₁₂ à C₂₄.

3. Composition selon la revendication 2, dans laquelle l'uréthane éthoxylé a R qui représente un groupe alkyle en C₁₈ et n se situe dans la plage de 75 à 100.

4. Composition selon l'une quelconque des revendications précédentes, comprenant en outre de 0,1 à 10 % en poids d'un polymère de taurate.

5. Composition selon la revendication 4, dans laquelle le polymère de taurate est l'acrylate d'hydroxyéthyle sodique/taurate d'acryloyldiméthyle sodique.

6. Composition selon la revendication 4, dans laquelle le polymère de taurate est l'acrylate de sodium/taurate d'acryloyldiméthyle sodique.

7. Composition selon l'une quelconque des revendications précédentes, comprenant en outre d'environ 0,1 à 5 % en poids de poly-isobutène hydrogéné.
